# EUROPÄISCHE PATENTSCHRIFT

(11) **EP 4 366 633 B1**
(45) Veröffentlichungstag und Bekanntmachung des Hinweises auf die Patenterteilung: **29.10.2025**
(21) Anmeldenummer: 22748022.5
(22) Anmeldetag: 08.07.2022
(51) Int. Cl.: A61B 17/30, G01N 33/00

(54) **VERFAHREN, VORRICHTUNG UND SYSTEM ZUR ENTFERNUNG DES CHORIONS EINES NICHT-MENSCHLICHEN EMBRYOS**
METHOD, DEVICE AND SYSTEM FOR REMOVING THE CHORION OF A NON-HUMAN EMBRYO
PROCÉDÉ, DISPOSITIF ET SYSTÈME POUR RETIRER LE CHORION D'UN EMBRYON NON-HUMAIN

(30) Priorität: 08.07.2021 DE 102021117617
(43) Veröffentlichungstag der Anmeldung: 15.05.2024
(73) Patentinhaber: Albert-Ludwigs-Universität Freiburg, 79098 Freiburg (DE)
(72) Erfinder: KAPP, Friedrich, 79117 Freiburg (DE); NOESKE, Harald, 79285 Ebringen (DE)
(74) Vertreter: Hertin und Partner Rechts- und Patentanwälte PartG mbB
(86) Internationale Anmeldenummer: PCT/EP2022/069113
(87) Internationale Veröffentlichungsnummer: WO 2023/281073

(56) Entgegenhaltungen:
- CULIC-VISKOTA JELENA ET AL: "Surface functionalization of barium titanate SHG nanoprobes for in vivo imaging in zebrafish", NATURE PROTOCOLS, NATURE PUBLISHING GROUP, GB, vol. 7, no. 9, 9 August 2012 (2012-08-09), pages 1618 - 1633, XP037655278, ISSN: 1754-2189, [retrieved on 20120809], DOI: 10.1038/NPROT.2012.087
- HENN K ET AL: "Dechorionation as a tool to improve the fish embryo toxicity test (FET) with the zebrafish (Danio rerio)", COMPARATIVE BIOCHEMISTRY AND PHYSIOLOGY PART C: TOXICOLOGY & PHARMACOLOGY, ELSEVIER, AMSTERDAM, NL, vol. 153, no. 1, 1 January 2011 (2011-01-01), pages 91 - 98, XP027534654, ISSN: 1532-0456, [retrieved on 20100930]

## Beschreibung

### BESCHREIBUNG

Die Erfindung betrifft ein Verfahren zur Entfernung des Chorions unter Bereitstellung einer Spreizpinzette, welche mindestens drei Spitzen umfasst, die endseitig an Pinzettenarmen vorliegen. Bei dem Verfahren erfolgt zunächst ein Durchstoßen des Chorions an den mindestens drei Punkten mittels der Spitzen sowie ein anschließendes Aufspreizen der Pinzettenarme, sodass sich zwischen den mindestens drei Punkten eine Öffnung, vorzugsweise eine polygonale Öffnung, in dem Chorion bildet, aus welcher der Embryo heraustreten kann. In einem weiteren Aspekt betrifft die Erfindung eine Spreizpinzette zur Durchführung des Verfahrens zur Entfernung des Chorions sowie ein System umfassend eine Spreizpinzette und einen Antrieb, welcher zur automatisierten Durchführung des Durchstoßens und Öffnen des Chorions konfiguriert ist. In bevorzugten Ausführungsformen wird das Verfahren zur Dechorionierung einer Vielzahl von Embryonen innerhalb eines Hochdurchsatz-Verfahrens, vorzugsweise zur automatisierten Testung der Wirkung von Substanzen auf Embryonen genutzt.

### Hintergrund und Stand der Technik

Die Erfindung betrifft Vorrichtungen, Systeme und Verfahren zur Dechorionierung von nicht menschlichen Embryonen, insbesondere Zebrafischembryonen, um ein sicheres, zügiges und vorzugsweise automatisiertes Entfernen der Embryonen aus deren Chorionhülle zur ermöglichen.

Die Erfindung findet insbesondere Anwendung in weitreichenden Gebieten, bei denen Embryonen, insbesondere Zebrafischembryonen, für die Grundlagen- oder medizinische Forschung genutzt werden.

Nach Pionierarbeiten, die in den 1970er Jahren von Georg Streisinger an der Universität von Oregon begonnen wurden, hat sich der Zebrafisch (*Danio rerio*) in den letzten 30 Jahren als wichtiger Modellorganismus für Wirbeltiere sowie zur Erforschung humaner Erkrankungen entwickelt (Dooley et al. 2000).

Der erfolgreiche Einsatz des Zebrafisches für die Grundlagen- oder medizinische Forschung beruht auf einer Reihe von Vorteilen.

Aufgrund der relativ geringen Größe von wenigen Zentimetern kann eine große Menge an adulten Fischen unter Laborbedingungen gehalten werden. Zebrafische lassen sich leicht vermehren, wobei die Weibchen bis zu 200 Eier legen und relativ kurze Generationszeiten von 3-4 Monaten besitzen.

Für die Untersuchung von Entwicklungsprozessen bzw. Substanzen, welche auf diese Einfluss nehmen können, sind Zebrafische besonders gut geeignet, da die Entwicklung von Zebrafischembryonen im Vergleich zu anderen Wirbeltieren sehr schnell ist und von der Befruchtung bis zum Schlüpfen einer schwimmenden Larve nur wenige Tage dauert.

Außerdem entwickeln sich die wenige Millimeter großen, durchsichtigen Embryonen extern und sind daher für eine Mikromanipulation, Mikroinjektion und Beobachtung leicht zugänglich.

Eine Fluoreszenzmarkierung von Proteinen oder Zellstrukturen kann durch die Injektion von mRNA, die für fluoreszierende Proteine kodiert, oder durch die Erzeugung von transgenen Reporterlinien erreicht werden. Darüber hinaus haben vorwärtsgenetische Screens eine große Sammlung von phänotypischen Mutanten generiert und die Identifizierung von Genen ermöglicht, die an der Entwicklung oder der Ausbildung von Krankheiten beteiligt sind. Eine gut annotierte Genomsequenz ist verfügbar und Anti-Sense-Morpholino-Oligonucletide können leicht zum Knock-down von Proteinen verwendet werden. Mittels Techniken der reversen Genetik, wie Transkriptionsaktivator-ähnliche Effektoren (TALEN), Zinkfingerähnliche Nukleasen (ZNFs) oder dem CRISPR-Cas-System ist ein Genome Editing im Zebrafisch möglich.

Besondere Bedeutung hat der Zebrafisch in der Medikamentenentwicklung aufgrund der Möglichkeit frühzeitiger, präklinischer Testung der Toxizität potentieller Wirkstoffe oder der Durchführung von Screening-Verfahren bei hohem Durchsatz erlangt.

Unvorhergesehene Nebenwirkungen in klinischen Studien für neue Medikamente sind in Bezug auf die gesundheitlichen Risiken der Studienteilnehmer und die finanziellen Kosten weitestgehend zu eliminieren. Präklinische Sicherheitstests sollen verhindern, dass toxische Wirkstoffe überhaupt in eine klinische Studie Eingang finden. Vorteilhaft kann in ZebrafischLarven eine aussagekräftige *in vivo*-Toxizitätsbewertung bereits innerhalb einer Woche erhalten werden, welches eine deutlich kürzeren Zeitrahmen darstellt, als dies für die Durchführung beispielsweise von Assays an Säugetieren, wie Mäuse, erforderlich ist. Das Zebrafischmodell stellt somit eine wichtige Brücke zwischen *in-vitro* Assays und in-vivo-Studien an Säugetieren dar und findet weitverbreitet Anwendung (Parng et al. 2002, Cassar et al. 2020).

Bei Screening-Verfahren mit dem Zebrafischmodell besteht die Möglichkeit eine große Anzahl an Medikamenten auf ihren phänotypischen Effekt auf die Zebrafischembryonen zu testen.

Phänotypbasiertes Screening zur Entdeckung von neuen Medikamenten wird in der biomedizinischen und pharmazeutischen Forschung zunehmend eingesetzt (Gehring et al. 2018). Im Gegensatz zum Target-basierten Screening erfordern phänotypbasierte Ansätze keine genaue Kenntnis des Targets. Darüber hinaus haben *in-vivo* Ansätze, welche auf der Beobachtung ganzen Organismen basieren, den Vorteil, dass sie toxische und andere Nebenwirkungen von Medikamenten in einem sehr frühen Stadium der Studie aufdecken können. In den letzten Jahren hat sich der Zebrafisch aufgrund seiner Vielseitigkeit und Leistungsfähigkeit als eines der wichtigstes Wirbeltier-Modellsystem für chemische Screening-Experimente mit hohem Durchsatz etabliert (MacRae et al. 2015, Rennekamp et al. 2015). Mindestens 10 klinische Kandidaten, welche in Zebrafischscreens entdeckt wurden, werden derzeit in klinischen Studien getestet (Cully et al. 2019).

Um eine präklinische Testung der Toxizität potentieller Wirkstoffe oder die Durchführung von Screening-Verfahren möglichst effizient zu gestalten, ist eine hohe Anzahl an Embryonen notwendig, sodass eine Automatisierung der Verfahrensschritte wünschenswert ist.

Im Stand der Technik sind zu diesem Zwecke verschiedene Ansätze bekannt.

Die US 2012/0178646 A1 offenbart beispielsweise eine Hochdurchsatzverfahren für ein *in vivo* Screening von Zebrafischembryonen, welches durch eine automatisierte Injektion gekennzeichnet ist. Zur Erhöhung des Durchsatzes werden hierbei bewusst Injektionsfehler in Kauf genommen werden, welche durch ein automatisiertes Sortierungssystem markiert bzw. aussortiert werden können.

Die WO 2019/032690 A1 schlägt einen Bioassay für Zebrafischembryonen vor, bei dem die Zebrafischembryonen innerhalb einer Fluidvorrichtung gezielt zwischen verschiedenen Kammern und Kanälen transportiert und immobilisiert werden können. Hierdurch soll auf eine Immobilisierung in separaten Agarosematrizen oder aber mittels Anästhetika verzichtet werden können und negative Einflüsse auf das Screening vermieden werden. Um das Chorion zur entfernen wird das Pronase-Enzyme eingesetzt.

Nachteilig kann die Verwendung von Pronase zur Verdauung des Chorions jedoch die Entwicklung der Zebrafischembryonen beeinflussen und die Mortalitätsraten erhöhen (vgl. Henn et al. 2011, Mandrell et al. 2012).

Henn et al. 2011 untersucht den Einfluss einer Dechorinierung von Zebrafischembryonen im Kontext einer Testung der Toxizität potentieller Wirkstoffe. Für die Entfernung des Chorions werden sowohl mechanische als auch pronasebasierte Verfahren verglichen. Insbesondere in frühen Stadien kann gemäß Henn et al. 2011 die Verwendung von Pronase zur Verdauung des Chorions die Entwicklung der Zebrafischembryonen beeinflussen und die Mortalitätsraten erhöhen. Für eine mechanische Dechorinierung konnten bessere Ergebnisse erzielt werden. Die mechanische Dechorinierung wird laborüblich mittels zweier Pinzetten durchgeführt.

Mandrell et al 2012 schlagen aufgrund des möglichen Einflusses von Pronase zur Verdauung des Chorions auf die Entwicklung der Zebrafischembryonen eine Kombination der Verwendung von Pronase mit einer automatisierten mechanischen Bewegung zur Dechorionierung. Die verbesserten Mortalitätsraten von weniger als 5% schließen einen residualen Einfluss der Dechorionierung mittels der Pronase auf die Embryonen weiterhin nicht aus.

In Forschungslaboren wird aufgrund eines möglichen Einflusses von Pronase auf die Entwicklung der Zebrafischembryonen und mithin den Forschungsergebnissen, oftmals eine manuelle Dechorionierung mittels Pinzetten vorgezogen.

Hierbei werden entweder zwei Pinzetten verwandt mit denen das Chorion ausgehend von zwei gegenüberliegenden Stellen auseinandergezogen wird. Alternativ wird eine Pinzette mit zwei Spitzen in das Chorion gestochen, wobei durch die Spreizung eine Öffnung geschaffen wird, durch welche der Embryo austreten kann. Eine laborübliche mechanische Dechorinierung von Zebrafischembryonen wird beispielsweise in Čulić-Viskota J. et al. 2012 beschrieben. So wird auf S. 1625 für die Vorbereitung der Embryonen für die Mikroskopie unter Punkt 40 A ii) die Verwendung scharfer Pinzetten offenbart, mit denen zunächst eine einzelnes Loch im Chorion geöffnet wird, durch den der Embryo vorsichtig austreten kann.

Nachteilig sind mögliche Verletzungen des Embryos während dieser durch die schlitzförmige Öffnung tritt. Das mechanische Dechorionieren mittels der Feinpinzetten bedarf daher ein hohes Maß an Übung und ist nicht in naheliegender Weise automatisierbar.

Im Lichte des Standes der Technik besteht mithin ein Bedarf an alternativen oder verbesserten Vorrichtungen oder Verfahren zur Entfernung des Chorions eines Embryos, insbesondere eines Zebrafischembryos.

### Aufgabe der Erfindung

Aufgabe der Erfindung ist es, ein Verfahren bzw. eine Vorrichtung bereitzustellen, welche die Nachteile des Standes der Technik beseitigen. Insbesondere war es eine Aufgabe der Erfindung, ein Verfahren zur Entfernung des Chorions eines Embryos bzw. eine dazu geeignete Vorrichtung bereitzustellen, welches auf einfache, schnelle und sichere Weise durchzuführen ist und sich zudem bevorzugt für eine Automatisierung eignet.

### Zusammenfassung der Erfindung

Die Aufgabe wird durch die Merkmale der unabhängigen Ansprüche gelöst. Bevorzugte Ausführungsformen der Erfindung sind in den abhängigen Ansprüchen beschrieben.

In einem ersten Aspekt betrifft die Erfindung bevorzugt ein Verfahren zur Entfernung des Chorions eines nicht menschlichen Embryos umfassend die Schritte
a) Bereitstellung einer Spreizpinzette umfassend mindestens drei Spitzen, welche endseitig an Pinzettenarmen vorliegen
b) Durchstoßen des Chorions an mindestens drei Punkten mittels der Spitzen
c) Aufspreizen der Pinzettenarme, sodass sich zwischen den mindestens drei Punkten eine Öffnung in dem Chorion bildet, aus welcher der Embryo heraustreten kann.

Das erfindungsgemäße Verfahren zeichnet sich dadurch aus, dass das Chorion der Embryonen mittel der Spreizpinzette an mindestens drei Punkten durchstoßen wird. Beim Öffnen bzw. Aufspreizen der Pinzettenarme der Spreizpinzette kann vorteilhaft hierdurch anstelle eines schmalen Schlitzes eine polygonale Öffnung gewährleistet werden, durch welche der Embryo unversehrt austreten kann.

Die im erfindungsgemäßen Verfahren verwendete Spreizpinzette umfasst zu diesem Zwecke mindestens drei Spitzen, welche endseitig an Pinzettenarmen installiert vorliegen.

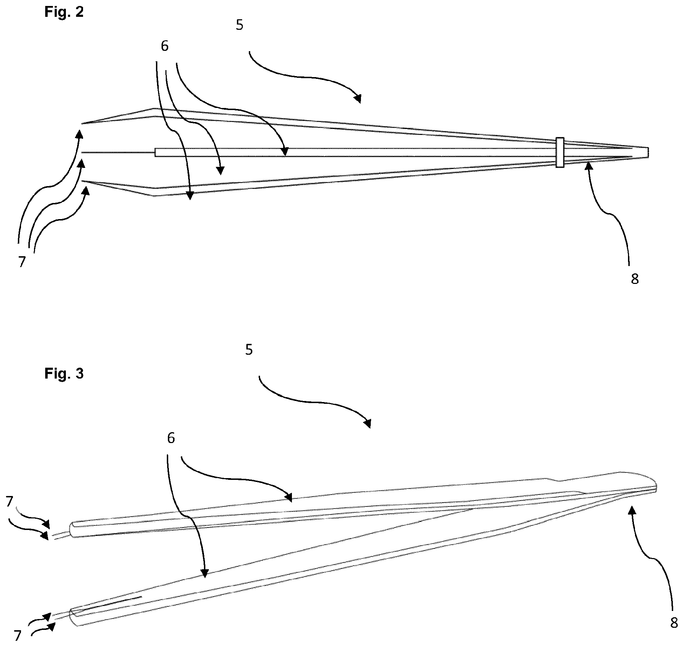

Hierbei kann es beispielsweise bevorzugt sein, drei oder mehr Pinzettenarme vorzusehen, an denen jeweils endseitig die Spitzen vorliegen (vgl. Fig. 2) und welche sich bei der Öffnung bevorzugt gleichmäßig auseinanderbewegen. Ebenso kann es bevorzugt sein, die Spreizpinzette mit nur zwei Pinzettenarmen auszugestalten, an denen zwei oder mehr Spitzen vorliegen (vgl. Fig. 3). Darüber hinaus kann es auch bevorzugt sein, beispielsweise eine Spreizpinzette mit drei oder mehr Pinzettenarmen bereitzustellen, wobei an mindestens einem der mindestens drei Pinzettenarme zwei oder mehr Spitzen endseitig vorliegen.

Von den Erfindern wurde erkannt, dass durch Bereitstellung einer Spreizpinzette mit drei oder mehr Spitzen der Prozess der Dechorionierung wesentlich einfacher, schneller und sicherer durchgeführt werden kann.

Durch das Durchstoßen des Chorions an mindestens drei Punkten kann zuverlässig die Bildung einer schmalen, schlitzförmigen Öffnung vermieden werden, welche mit bisherigen Mitteln eine Gefahr zur Quetschung von Embryonen darstellt.

Ein derartiger Nachteil besteht beispielsweise bei bekannten Pinzetten mit zwei Spitzen zur Bildung einer Öffnung. Mittels einer derartigen und üblichen Pinzette muss ein überaus vorsichtiges Öffnen erfolgen, um zu vermeiden, dass der Embryo durch den sich schmal öffnenden Schlitz herausgequetscht und hierbei verletzt wird, was unweigerlich zum Tod des Embryonen führt.

Daher wird für die manuelle Dechorionierung von Embryonen, insbesondere Zebrafischembryonen, Greifpinzetten verwendet. Hierbei werden mittels zweier separater Greifpinzetten das Chorion des Embryos an zwei Punkten vorsichtig auseinander gezogen, um eine sich langsam öffnende, schlitzförmige Öffnung zu erhalten, wobei auch diese Öffnung meist noch zu klein ist, und das Chorion am Riss entlang nachgegriffen werden muss, um den Vorgang zu kontrollieren. Auch bei diesem bekannten Verfahren muss das Auseinanderziehen behutsam erfolgen, um ein schnelles Austreten und Quetschungen oder Verletzungen des Embryos zu verhindern. Für geübtes Personal ist es möglich das Chorion an verschiedenen Punkten zu öffnen, um sukzessive eine sich vergrößernde Öffnung zum Austritt des Embryos bereitzustellen.

Den bekannten, mechanischen Verfahren zum Entfernen des Chorions ist gemein, dass diese viel Übung erfordern, um in Bezug auf die Fehlerquote vernünftige Ergebnisse zu erhalten. Doch selbst für sehr geübtes Fachpersonal ist der Prozess mühselig, zeitaufwändig und Bedarf hoher Konzentration.

Das erfindungsgemäße Verfahren ist demgegenüber äußert zeiteffizient, vermeidet Verletzung von Embryonen und kann auch von unerfahrenem Mitarbeiten nach wenigen Übungen durchgeführt werden.

Wie hierin noch weiter im Detail beschrieben, ist das erfindungsgemäße Verfahren zudem vorteilhaft einer Automatisierung zugänglich, um Embryonen mit hohem Durchsatz zu dechorionieren. Eine derartige Automatisierung war für die bekannten mechanischer Verfahren zum Entfernen des Chorions nicht denkbar.

Der Begriff "dechorionieren" meint im Sinne der Erfindung bevorzugt die Befreiung eines Embryos aus seinem Chorion. Chorion bezeichnet bevorzugt die Eihülle insbesondere von Fischeiern. Im Gegensatz zu Säugetieren entwickeln sich die Fischembryonen außerhalb des Muttertiers extern. Das Chorion dient als Schutzhülle vor extern Einflüssen bis der Embryo robust genug ist, um diesen zu widerstehen. Zebrafischlarven schlüpfen in der Regel nach ca. drei Tagen aus dem Chorion.

Soweit nicht anders benannte soll unter dem Begriff Embryo ein Organismus in frühen Entwicklungsstadien verstanden werden, insbesondere sofern sich der entwickelnde Embryo noch in einem Chorion befinden. Für den Zebrafisch werden die Entwicklungsstadien ab der Befruchtung der Eizelle bis ca. 48 oder 72 h pf (*post fertilization*) als Embryonalphase bezeichnet, während nach dem Schlüpfen die Embryonen als Larven bezeichnet werden. Im Sinne der Erfindung kann es jedoch bevorzugt sein, dass in Abhängigkeit unter dem Begriff Zebrafischembryo auch Zebrafischlarven verstanden, werden können.

Bei der Verwendung von Zebrafischen in der Grundlagen- oder medizinischen Forschung ist es zweckmäßig das Chorion vor dem natürlichen Schlüpfen zu entfernen.

Insbesondere für Verfahren der Mikromanipulation oder zur mikroskopischen Untersuchung der Embryonen ist das Chorion hinderlich und muss entfernt werden. Die Bildgebung durch ein vorhandenes Chorion ist zum einen aufgrund der Absorption häufig von proteinreichem Material in der Chorionflüssigkeit komprimiert. Zum anderen lassen sich die im Chorion befindlichen Embryonen nicht immobilisieren, so dass eine Beobachtung von bestimmten Strukturen im Mikrometer-Bereich nicht möglich sind.

Aufgrund der potentiellen Barrierewirkung des Chorions sollte auch im Falle präklinischer Testung der Toxizität potentieller Wirkstoffe oder für die Durchführung phänotypischer Screening-Verfahren das Chorion entfernt werden, um einen Einfluss auf die Testergebnisse auszuschließen.

Vorteilhaft lässt sich das erfindungsgemäße Verfahren auf eine Vielzahl von Embryonen anwenden, welche sich extern in einem Chorion entwickeln.

In einer bevorzugten Ausführungsform der Erfindung wird das erfindungsgemäße Verfahren zur Entfernung eines Chorions eines Embryos genutzt, wobei der Embryo ein Fischembryo ist, bevorzugt ein Zebrafischembryo (Zebrabärbling, *Danio rerio*), ein Killifischembryo (Eierlegenden Zahnkarpfen) oder Medakaembryo (Japanischer Reisfisch) ist.

Zebrafisch-, Killifisch- oder Medakaembryonen finden verbreitet Einsatz als Modellorganismen in der Grundlagenforschung oder im Rahmen medizinischer Testverfahren. Vorteilhaft führt die Bereitstellung einer Spreizpinzette mit mindestens drei Spitzen zur Bildung einer Öffnung zwischen mindestens drei Punkten bei sämtlich vorgenannten Modellorganismen zu den erfindungsgemäßen Vorteilen im Hinblick auf eine erhöhte Zeiteffizienz sowie einer Vermeidung von unerwünschten Verletzungen der Embryonen.

Die Durchführung des Verfahrens der Dechorionierung kann zu jedem Zeitpunkt der Entwicklung eines Embryos erfolgen sofern sich dieser noch im Chorion befindet. Für Zebrafischembryonen kann beispielsweise eine Dechorionierung bevorzugt innerhalb der ersten 72h, 48 h, 24 h, 10 h oder 5 h nach der Befruchtung oder auch direkt nach der Befruchtung erfolgen.

In einer bevorzugten Ausführungsform ist das Verfahren dadurch gekennzeichnet, dass sich die mindestens drei Punkte, an denen das Chorion durchstoßen wird nicht auf einer Linie liegen, sondern ein Polygon aufspannen. Die drei Punkte liegen mithin bevorzugt bereits beim Durchstoßen des Chorions auf nicht kollinearen Punkten vor. Hierdurch kann bei dem folgenden Aufspreizen die Ausbildung einer polygonalen Öffnung gewährleistet werden, welche den Austritt der zumeist sphärischen Embryonen erleichtert und deutlich sicherer gestaltet.

Bei dem Polygon kann es sich beispielsweise um ein Dreieck (Trigon), Viereck (Tetragon), Fünfeck (Pentagon), Sechseck (Hexagon), Siebeneck (Heptagon), Achteck (Oktogon), Neuneck (Nonagon) oder Zehneck (Dekagon) handeln. Ebenso können auch Vielecke mit einer höheren Anzahl von 10 oder mehr Ecken bevorzugt sein.

In einer bevorzugten Ausführungsform umfasst die Spreizpinzette mindestens drei Pinzettenarme, an welchen jeweils endseitige Spitzen vorliegen, und wobei das Chorion an mindestens drei Punkten durchstoßen wird, welche ein Dreieck bilden, bevorzugt ein im Wesentlichen gleichseitiges Dreieck.

Begriffe wie im Wesentlichen, ungefähr, etwa, ca. etc. beschreiben bevorzugt einen Toleranzbereich von weniger als ± 20%, bevorzugt weniger als ± 10%, besonders bevorzugt weniger als ± 5 %, noch stärker bevorzugt weniger als ± 3% und insbesondere weniger als ± 1%, wobei stets auch der exakte Wert umfasst sein kann.

Eine bevorzugte Ausführungsform mit drei Pinzettenarme an denen endseitige Spitzen vorliegen, wird beispielhaft in der Fig. 2 illustriert. Bevorzugt sind die drei Pinzettenarme und die endseitigen Spitzen derart angeordnet, dass diese vor dem Aufspreizen im Wesentlichen in einer Ebene vorliegen, um ein gleichzeitiges Durchstoßen des Chorions an drei Punkten zu gewährleisten. Bei der Aufspreizbewegung ist es bevorzugt, dass alle Pinzettenarme oder mindestens zwei der drei Pinzettenarme auseinander gespreizt werden, um eine Öffnung eines Dreiecks zu ermöglichen. Im Falle eines Polygons mit mehr als drei Ecken, beispielsweise eines Polygon mit vier, fünf, sechs oder mehr Ecken, kann es bevorzugt sein, eine entsprechende Anzahl von Pinzettenarmen mit jeweils einer endseitigen Spitze bereitzustellen.

In bevorzugten Ausführungsformen kann die Spreizpinzette zwei, drei, vier, fünf, sechs oder mehr Pinzettenarme aufweisen, auf denen endseitig jeweils eine Spitze positioniert vorliegt. Vorzugsweise werden alle oder alle bis auf einen Pinzettenarm aufgespreizt, um eine polygonale Öffnung zu erhalten.

In einer weiteren bevorzugten Ausführungsform des Verfahrens ist die Spreizpinzette dadurch gekennzeichnet, dass diese mindestens zwei Pinzettenarme umfasst an denen endseitig jeweils mindestens zwei Spitzen vorliegen und das Chorion an mindestens vier Punkten durchstoßen wird, welche bevorzugt ein Viereck bilden und wobei bevorzugt durch das Aufspreizen der zwei Pinzettenarme eine Länge des Viereckes vergrößert wird.

Eine bevorzugte Ausführungsform mit zwei Pinzettenarmen an denen endseitig jeweils zwei Spitzen vorliegen wird beispielhaft in der Fig. 3 illustriert. Bevorzugt sind die zwei Pinzettenarme und die vier endseitigen Spitzen derart angeordnet, dass diese vor dem Aufspreizen im Wesentlichen in einer Ebene vorliegen, um ein gleichzeitiges Durchstoßen des Chorions an vier Punkten zu gewährleisten. Bei der Aufspreizbewegung ist es bevorzugt, dass beide Pinzettenarme oder mindestens einer der beiden Pinzettenarme aufgespreizt wird, um eine Öffnung eines Vierecks zu bewirken. Durch den Abstand zweier Spitzen auf den jeweiligen Pinzettenarme kann vorzugsweise eine erste Länge des sich öffnenden Vierecks festgelegt sein, während sich eine zweite Länge im Zuge der Aufspreizbewegung vergrößert.

In bevorzugten Ausführungsformen kann die Spreizpinzette zwei, drei, vier, fünf oder Pinzettenarme aufweisen, auf denen endseitig jeweils zwei, drei, vier, fünf Spitzen positioniert vorliegen, um das Chorion an einer Mehrzahl der Ecken, vorzugsweise im Wesentlichen gleichzeitig zu durchstoßen.

Durch Bereitstellung einer Vielzahl von Spitzen kann es bevorzugt sein, eine polygonale Öffnung mit einer Vielzahl von Ecken, beispielsweise sechs, acht oder mehr Ecken bereitzustellen, welche den Austritt der häufig sphärischen Embryonen noch stärker erleichtert.

In einer bevorzugten Ausführungsform der Erfindung ist das Verfahren dadurch gekennzeichnet, dass die Pinzettenarme automatisiert geführt werden, wobei insbesondere das Durchstoßen des Chorions und/oder Aufspreizen der Pinzettenarme zur Entfernung des Chorions automatisiert erfolgt.

Automatisiert meint bevorzugt, dass die Bewegung der Pinzettenarme mittels eines Antriebs motorisiert und insbesondere ohne manuelle Betätigung erfolgen kann. Stattdessen ist es bevorzugt, dass das Führen der Pinzettenarme mittels einer Steuereinrichtung, beispielsweise eines Computers, auf Basis entsprechender Steuerbefehle automatisiert bzw. motorisiert erfolgt.

Zu diesem Zweck kann es beispielsweise bevorzugt sein, dass die Pinzettenarme über separate Antriebe translatiert und/oder rotiert werden können.

Besonders bevorzugt erfolgt zunächst eine Bewegung der Pinzettenarmen mit den endseitig positionierten Spitzen in eine erste Position, in welche das Chorion an drei oder mehr Punkten durchstochen wird. Im Anschluss erfolgt eine Bewegung bzw. ein Aufspreizen einer oder mehrerer der mindestens zwei Pinzettenarme in eine zweite Position, wodurch es wie beschrieben, zur Bereitstellung einer polygonalen Öffnung kommt.

Während der Dechorionierung ist es bevorzugt, dass das Chorion mit Embryo zu einer Spreizpinzette ausgerichtet vorliegt, vorzugsweise im Wesentlichen immobilisiert in Bezug auf mindestens eine Translationsrichtung.

Beispielsweise können einzelnen Embryonen im Chorion in einer Halte- oder Positioniervorrichtung in einer linienförmigen Vertiefung (oder auch Rinne, Furche, Kerbe) aufgereiht vorliegen, wobei eine Spreizpinzette die Embryonen einzeln nacheinander automatisiert dechorioniert. Eine hintere Wand der linienförmigen Vertiefung kann beispielsweise einem Weggleiten des Chorions bei Vollzug einer Durchstoßbewegung der Spitzen in die erste Position wirksam entgegenstehen.

Ebenso kann es beispielsweise auch bevorzugt sein, die Embryonen im Chorion in Kompartimenten oder Mulden zu positionieren, um eine Aussichtung mit der Spreizpinzette zur ermöglichen. Bevorzugt können sich die Embryonen beispielsweise auch in den Zwischenräumen eines rotierbaren Zahnrad (oder Drehkreuzes) vor einer ortsfesten Spreizpinzette befinden. Auch eine Positionierung auf einem flachen Substrat als Positioniervorrichtung ist möglich, wobei die initiale Bewegung zum Durchstoßen des Chorions vorzugsweise im Wesentlichen senkrecht von oben erfolgt, um eine Ausweichbewegung zu verhindern.

Für einen Übergang der Bearbeitung eines Embryos zum nächsten Embryo kann es bevorzugt sein, den Embryo bzw. eine Positioniervorrichtung oder Haltevorrichtung auf dem dieser vorliegt, und/oder die Spreizpinzette zu bewegen.

Die Ausrichtung der Embryonen zur Spreizpinzette oder umgekehrt, kann durch die Form der Positioniervorrichtung gewährleistet werden (z.B. durch Mulden, linienförmigen Vertiefungen, Zahnrad etc.). Auch eine visuelle Kontrolle, beispielsweise mittels Kamera und automatisierter Bildanalyse zur Überwachung der Position der Embryonen, ist möglich.

Im Gegensatz zu bisherigen mechanischen Ansätzen zur Dechorionierung, beispielsweise mittels zweier separater Greifpinzetten, lässt sich das erfindungsgemäße Verfahren deutlicher einfacher und robuster automatisieren. Zur Abbildung eines Verfahrens zur Dechorionierung mittels zweier Greifpinzetten, ist beispielsweise eine sehr aufwändige fein motorische Steuerung der Greifpinzetten notwendig, welche zudem neben einer Translation auch Greifbewegungen motorisiert durchführen müssen und Informationen über den Öffnungsprozess so verarbeitet, dass auf ein zu schnelles Einreißen des Chorions automatisiert reagiert wird.

Demgegenüber kann das erfindungsgemäße Verfahren vorteilhaft durch einfache Translationsbewegungen der Pinzettenarme zuverlässig umgesetzt werden, ohne dass die Embryonen Schaden nehmen und ohne, dass der Vorgang so überwacht werden muss, dass eine programmierte Antwort des Computers in die Dechorionierung eingreifen muss.

Die Automatisierung des Verfahrens führt zu ausgezeichneten Ergebnissen in Bezug auf Geschwindigkeit, wie auch einer Gewährleistung der Unversehrtheit von Embryonen.

Demnach können mittels des automatisierten Verfahrens vorzugsweise mehr als 100, 200, 300, 400, 500 oder mehr Embryonen pro Stunde zuverlässig auf mechanischem Wege vom Chorion befreit werden.

Ein derart hoher Durchsatz von Embryonen ist bisher nur mit chemischen Verfahren zur Degradation des Chorions bekannt, beispielsweise unter Verwendung von Pronase.

Hierbei kann es jedoch - wie eingangs ausgeführt - zu Störungen der Entwicklung kommen (vgl. Henn et al. 2011, Mandrell et al. 2012), welche die Testergebnisse verfälschen können.

Das automatisierte Verfahren zur Entfernung des Chorions kann vorzugsweise in Testverfahren zum Einsatz kommen, bei denen einer hoher Durchsatz von Embryonen erforderlich ist, wobei insbesondere auf chemische oder enzymatische Mittel zur Degradation des Chorions verzichtet werden kann.

In einem weiteren Aspekt betrifft die Erfindung daher ein Hochdurchsatz-Verfahren zur automatisierten Testung der Wirkung von Substanzen auf eine Vielzahl von nicht menschlichen Embryonen, bevorzugt Zebrafisch- oder Medakaembryonen, wobei eine Entfernung des Chorions der Embryonen mittels des beschriebenen Verfahrens erfolgt.

Ein Hochdurchsatz-Verfahren meint vorzugsweise ein Verfahren, welches eine Testung der Wirkung von Substanzen auf eine Vielzahl von Embryonen, vorzugsweise von mehr als 100, 500, 1000, 5000 oder mehr Embryonen betrifft, wobei bevorzugt der Schritt des Dechorionierens der Vielzahl von Embryonen weniger als 12 h, 6 h, 4h, 2h oder 1 h bedarf.

Bei dem Hochdurchsatz-Verfahren kann es sich vorzugsweise um ein Screening-Verfahren, insbesondere zur Entdeckung neuer Medikamente handeln, wobei die sich insbesondere Zebrafisch als geeignete Modellorganismen anbieten.

Bei Screening-Verfahren mit dem Zebrafisch-Modell besteht die Möglichkeit eine große Anzahl an potentiellen Wirkstoffen oder Medikamenten auf ihren Effekt auf einer Vielzahl von Zebrafischembryonen zu testen. Insbesondere ist dies nützlich, wenn Krankheitsmodelle menschlicher Erkrankungen im Zebrafisch (z.B. für Leukämie) verwendet werden, um neue Medikamente zu identifizieren. Um dies möglichst effizient zu gestalten, ist eine hohe Anzahl an Embryonen notwendig, was bei manueller Dechorionierung jedoch sehr zeitintensiv ist. Durch einer automatisierten Dechorionierung, beispielsweise mittels einer computergesteuerten Kamerakontrolle, kann die Verfügbarkeit von einer Vielzahl von Zebrafischembryonen gesichert werden bei gleichzeitiger Entlastung der Labormitarbeiter.

Vorzugsweise ist die automatisierte Durchführung der Dechorionierung in den HochdurchSatzverfahren ein Schritt im einem mehrstufigen automatisierten Verfahren, welches zudem eine automatisierten Sortierung und automatische Positionierung der Embryonen zur Vornahme der sowie eine anschließende Qualitätsbeurteilung, ggf. Sortierung in Screening-Platten sowie Behandlung (Testung der Wirkung von Substanzen) und deren Auswertung. Weitere Vorzugsvarianten, wie z.B. ein automatisierter Transfer der Embryonen in die Testlösungen können die Screening-Abläufe weiter beschleunigen.

Das Hochdurchsatz-Verfahren kann vorteilhaft auch die Testung der Toxizität eines oder mehrerer Wirkstoffe an einer Vielzahl von Embryonen betreffen. Insbesondere können hierdurch potentielle toxische Wirkungen in einem präklinischen Test, frühzeitig erkannt werden, sodass verhindert wird, dass die Wirkstoffe in klinische Studie Eingang finden. Mittels Zebrafischembryonen - bzw. -larven können aussagekräftige *in vivo*-Bewertung der Toxizität vorteilhaft bereits innerhalb weniger Tage erhalten werden, was einen bedeutenden Zeitvorteil gegenüber anderen Modellorganismen, wie beispielsweise Mäusen bedeutet.

Im Kontext der Hochdurchsatz-Verfahren erlaubt das erfindungsgemäße Verfahren zur Dechorionierung erstmal ein mechanisches Verfahren zur Entfernung des Chorions, welche gänzlich auf den Einsatz von chemischen Mitteln oder Enzymen, wie beispielsweise Pronase verzichten und dennoch eine Vielzahl von dechorionierten Embryonen innerhalb kurzer Zeit bereitstellen kann.

In einem weiteren Aspekt betrifft die Erfindung eine Spreizpinzette für ein beschriebenes Verfahren zur Entfernung eines Chorions umfassend mindestens drei Spitzen, welche endseitig an Pinzettenarmen vorliegen, wobei die Pinzettenarme für eine Bewegung in mindestens zwei Positionen konfiguriert sind, sodass die mindestens drei Spitzen in einer ersten Position ein Chorion in mindestens drei Punkten durchstoßen können und wobei ferner durch ein Aufspreizen der Pinzettenarme in eine zweite Position, sich zwischen den mindestens drei Punkten eine Öffnung in dem Chorion bildet, aus welcher der Embryo heraustreten kann.

Der durchschnittliche Fachmann erkennt, dass technische Merkmale, Definitionen und Vorteile bevorzugter Ausführungsformen, welche für das erfindungsgemäßen Verfahren offenbart wurden, gleichermaßen für die Spreizpinzette gelten und umgekehrt.

Die Pinzettenarme der Spreizpinzette dienen bevorzugt zum Führen der Spitzen, mit denen das Chorion an drei oder mehr Punkten durchstoßen und geöffnet werden kann. Die Spitzen sind vorzugsweise durch eine Verjüngung und/oder endseitig kleinen Querschnitt von vorzugsweise 0,1 mm², 0,01 mm² oder weniger gekennzeichnet, um das Durchstoßen des Chorions zu erleichtern. Die Pinzettenarme hingegen können durchaus stärker und stabiler ausgeführt sein.

Die Spitzen können hierbei als separate Strukturen auf einen Pinzettenarmen aufgesetzt vorliegen (vgl. Fig. 3). Es kann aber auch bevorzugt sein, dass die Pinzettenarme an jeweils einem Ende durch eine Verjüngung in funktionellen Spitzen übergehen (vgl. Fig. 2).

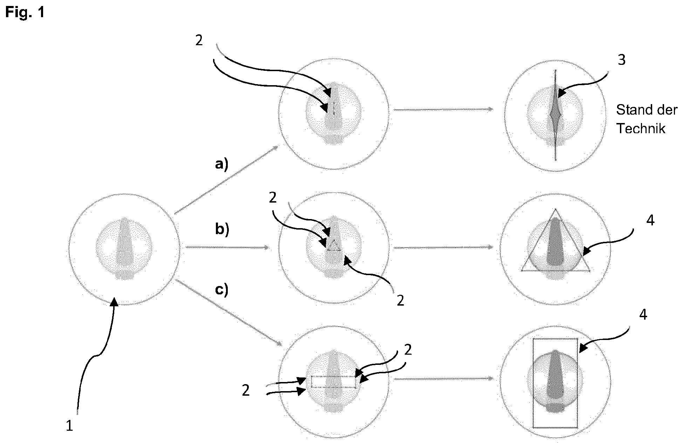

Das Durchstoßen des Chorions erfolgt bevorzugt in einer ersten Position an mindestens drei Punkten mittels der durch die Pinzettenarme geführten Spitzen. Ein Aufspreizen meint bevorzugt die Bewegung bzw. Beugung der Pinzettenarme, sowie der daran endseitig vorliegenden Spitzen von einer ersten Position in eine zweite Position, wodurch eine polygonale Öffnung erhalten werden kann, deren Anzahl von Ecken der Anzahl von Spitzen entspricht (vgl. Fig. 1).

In einer bevorzugten Ausführungsform umfasst die Spreizpinzette mindestens drei Pinzettenarme, an welchen jeweils eine endseitige Spitze vorliegt.

In einer weiteren bevorzugten Ausführungsform umfasst die Spreizpinzette mindestens zwei Pinzettenarme umfasst, an welchen jeweils zwei endseitige Spitzen vorliegen.

In einer weiteren bevorzugten Ausführungsform sind die Pinzettenarme elastisch federnde Schenkel, welche an ihrem einen Ende starr verbunden sind, wobei Pinzettenarme durch Ausübung eines Druckes in die erste Position gebracht werden können und bei Entfernung des Druckes elastisch federnd eine Aufspreizbewegung in die zweite Position vollziehen.

Die Ausführungsform ist besonders geeignet für eine manuelle Nutzung des Spreizpinzette. Ein Nutzer der Spreizpinzette kann durch Ausübung eines Druckes auf die Pinzettenarme diese in eine erste Position bringen, wobei vorzugsweise die Spitzen aufeinander zu geführt werden. Nach einem Durchstoßen des Chorions mit den Spitzen in der ersten Position, erfolgt das Öffnen bzw. Aufspreizen vorteilhaft kontrolliert zu einer vordefinierten zwei Position aufgrund der elastischen Eigenschaften der Pinzettenarme. Die zweite Position entspricht vorzugsweise einer kräftefreien, Ruheposition der Pinzettenarme (sowie der daran endseitig positionierten Spitzen).

Zur Bereitstellung elastisch federnder Schenkel, können die Pinzettenarme aus einem Metall gebildet werden und an einem Ende starr verbunden, vorzugsweise unter einem Einschlusswinkel von größer 0° vorliegen. Der Einschlusswinkel der elastisch federnden Schenkel am starren Ende, sowie deren Länge, definiert vorzugsweise die zweite Position der Spitzen und mithin die Dimension der nach dem Aufspreizen erreichten polygonalen Öffnung.

Die gewünschte polygonale Öffnung kann vorzugsweise an die zu dechorionierenden Embryonen angepasst werden. Für Zebrafischembryonen mit einem initialen Durchmesser von ca. 0,7 bis 0,8 mm, kann es bevorzugt seine eine polygonale Öffnung in der zweiten Position von mehr als 0,5 mm², vorzugsweise mehr als 1 mm², 2 mm² oder mehr zu gewährleisten.

In einer bevorzugten Ausführungsform bilden die Spitzen in der zweiten Position eine polygonale Öffnung mit mehr als 0,5 mm², vorzugsweise mehr als 1 mm², 2 mm² aber weniger als 50 mm², 20 mm² oder 10 mm².

An dem von den Spitzen abgewandten Ende, liegen die zwei oder mehr Pinzettenarme starr verbunden vor. Zu dem Zweck können die Pinzettenarme verklebt an dem von den Spitzen abgewandten Ende vorliegen. Ebenso können die Pinzettenarme jedoch auch endseitig verschraubt oder geklemmt werden. Auch eine monolithische Bildung der Pinzettenarme als federnde Schenkel, welche endseitig starr verbunden vorliegen, ist möglich.

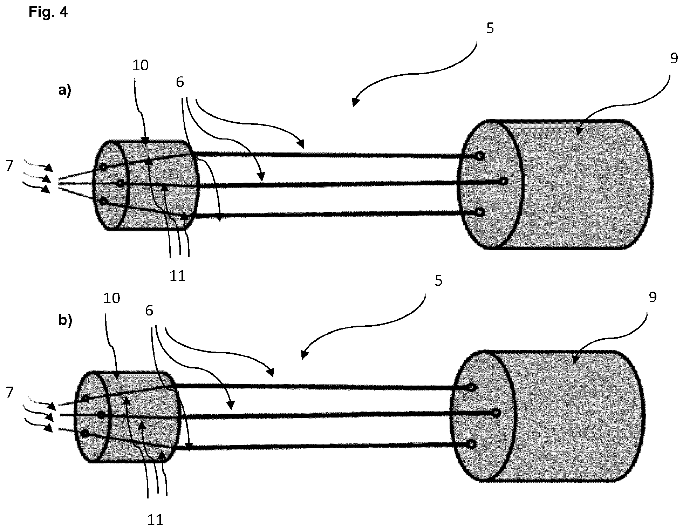

In bevorzugten Ausführungsformen ist die Spreizpinzette dafür eingerichtet, dass die Öffnungsbewegung der Pinzettenarme im gleichen Maße erfolgt. Hierfür wird bevorzugt ein Mechanismus bereitgestellt, bei dem die Biegung und Spannung der Pinzettenarme durch den Durchtritt durch leicht gewinkelte Löcher im Öffnungsmechanismus bewirkt werden kann. Fig. 4 illustriert einen bevorzugten Mechanismus zur Gewährleistung einer gleichmäßigen Öffnung.

In einer bevorzugten Ausführungsform liegen die Pinzettenarme endseitig in einem Griff stabilisiert vor und werden in einem Zwischenstück mit Führungsbohrungen auf eine Zentralachse des Zwischenstückes zugeführt, sodass durch eine Vergrößerung des Abstandes zwischen Zwischenstückes und Griff die Pinzettenarme von einer ersten Position zu einer zweiten Position aufgespreizt werden.

Am Eintrittsende des Zwischenstückes sind die Führungsbohrungen vorzugsweise kongruent zu jenen Positionen im Griff angeordnet, in welchen die Pinzettenarme stabilisiert werden, sodass die Pinzettenarme zwischen dem Griff und dem Zwischenstück vorzugsweise im Wesentlichen parallel verlaufen. Innerhalb des Zwischenstückes verlaufen die Führungsbohrungen vorzugsweise hingegen von einer äußeren radialen Position am Eintrittsende zu einer inneren radialen Position, sodass die Pinzettenarme innerhalb des Zwischenstückes in Richtung der Zentralachse geführt bzw. gebogen werden. Die Beabstandung der Führungsbohrungen von der Zentralachse am Austrittsende kennzeichnet vorzugsweise die maximale Aufspreizposition der Pinzettenspitzen. Wird der Abstand des Zwischenstückes zum Griff derart vergrößert, dass die Pinzettenarme bzw. Pinzettenspitzen nur geringfügig aus den Führungsbohrungen am Austrittsende vorstehen, liegen die Pinzettenspitzen in einer maximalen Aufspreizposition vor, welche durch die Führungsbohrungen am Austrittsende des Zwischenstückes vorgegeben ist. Durch eine Verringerung des Abstandes zwischen dem Zwischenstück und dem Griff können die Pinzettenspitzen aufeinander zugeführt werden, vorzugsweise bis zu einer ersten Position in welcher die Pinzettenspitzen sich nahezu berühren. Bevorzugt erfolgt in dieser Position ein Durchstoßen des Chorions an mindestens drei Punkten. Durch Verschiebung des Zwischenstückes in eine zweite Position unter Vergrößerung des Abstandes werden die Pinzettenspritzen aufgespreizt, sodass im Chorion ein polygonale Öffnung gebildet wird, durch welche der Embryo unversehrt austreten kann.

Vorteilhaft kann durch die Führungsbohrungen hierbei gewährleistet werden, dass die Aufspreizbewegung besonders gleichmäßig erfolgt, wodurch das Risiko einer Verzerrung der Öffnung oder Verletzung des Embryonen nochmals reduziert wird.

In einer bevorzugten Ausführungsform der Spreizpinzette sind die mindestens drei endseitigen Spitzen austauschbar ausgeführt. Vorzugsweise können die endseitigen Spitzen und/oder Pinzettenarme, an welchen die endseitigen Spitzen vorliegen, zu diesem Zweck einen Adapter aufweisen, welcher einen Austausch ermöglich. Ein solcher Adapter kann beispielsweise ein sogenannter Luer-Adapter (bzw. ein Luer/Slip-Adapter oder auch Steckverbindung), welcher bekanntermaßen für einen Austausch von medizinischen Kanülen eingesetzt wird oder ein Adapter, welcher eine ähnliche Funktion ermöglicht. In der Ausführungsformen sind die die endseitigen Spitzen und/oder Pinzettenarme, an welchen die endseitigen Spitzen vorliegen, vorzugsweise unterbrochen in mindestens zwei Teilbereiche ausgebildet, welche durch vorzugsweise einen Adapter miteinander verbunden werden. Dies ermöglich den gezielten Austausch der endseitigen Spitzen, sofern diese beispielsweise durch Verschleiß stumpf geworden sind oder andere Beeinträchtigungen, wie beispielsweise Verbiegungen aufweisen.

Hierdurch kann stets gewährleistet werden, dass für eine Dechorionierung optimal geformte und funktionstüchtige endseitige Spitzen bereitgestellt, ohne dass der Austausch der gesamten Spreizpinzette nötig wäre. Die bevorzugte Ausführungsform verbessert mithin sowohl die Funktionsfähigkeit, als auch Wirtschaftlichkeit der vorgeschlagenen Spreizpinzette.

In Bezug auf obiges Beispiel einer Spreizpinzette, bei der die Pinzettenarme endseitig in einem Griff stabilisiert vorliegen und in einem Zwischenstück mit Führungsbohrungen auf eine Zentralachse des Zwischenstückes zugeführt werden (vgl. auch Fig. 4) ist es bevorzugt den Adapter in einem Bereich der jeweiligen endseitigen Spitzen einzubringen, nach dem die endseitigen Spitzen aus dem Zwischenstück austreten. Durch die mithin erreichte Austauschbarkeit der endseitigen Spitzen bzw. deren Enden kann die Spreizpinzette umfassend Griff, Zwischenstück als auch Pinzettenarme besonders wirtschaftlich genutzt werden. Im Falle von Verschleiß, Bruch oder Verformung der endseitigen Spitzen, können diese gezielt ersetzt werden, ohne dass ein Austausch oder Reparatur der restlichen Komponenten der Spreizpinzette nötig wäre.

In einem weiteren Aspekt betrifft die Erfindung ein System umfassend
a. eine Spreizpinzette gemäß der Erfindung oder einer bevorzugten Ausführungsform hiervon
   sowie
b. einen Antrieb zur Bewegung der Pinzettenarme
wobei der Antrieb mindestens für eine Bewegung der Pinzettenarme zwischen einer ersten und einer zweiten Position konfiguriert ist.

Der durchschnittliche Fachmann erkennt, dass technische Merkmale, Definitionen und Vorteile bevorzugter Ausführungsformen, welche für das erfindungsgemäßen Verfahren oder die Spreizpinzette offenbart wurden, gleichermaßen für das System gelten und umgekehrt.

Das erfindungsgemäße System ist insbesondere für eine automatisierte Durchführung einer Dechorionierung von Embryonen eingerichtet und geeignet.

Zu diesem Zweck kann mittels eines vorzugsweise motorisierten Antriebes eine Bewegung der Pinzettenarme in eine erste Position zum Durchstoßen des Chorion an drei oder mehr Punkten erfolgen. Anschließend erfolgt vorzugsweise ein Aufspreizen der Spitzen durch Bewegung der Pinzettenarme in eine zweite Position, sodass sich wie beschrieben eine polygonale Öffnung bildet.

Als Antrieb kann beispielsweise ein elektrischer, pneumatischer oder hydraulischer Antrieb fungieren, welcher bevorzugt mittels einer Steuereinheit reguliert werden kann.

In einer bevorzugten Ausführungsform umfasst das System eine Steuereinheit zur Regulation des Antriebs, wobei die Steuereinheit bevorzugt für eine wiederholte Ausführung einer Bewegung der Pinzettenarme zum Durchstoßen des Chorions mittels der Spitzen an mindestens drei Punkten in einer ersten Position und einem Aufspreizen der Pinzettenarme in eine zweite Position zur wiederholten Entfernung des Chorions einer Vielzahl von Embryonen konfiguriert ist.

Wie hier verwendet, bezieht sich die "Steuereinheit" vorzugsweise auf ein beliebiges Computergerät oder -system mit einem Prozessor, einem Prozessorchip, einem Mikroprozessor oder einem Mikrocontroller, um eine automatische Steuerung der Spreizpinzette oder weitere Komponenten (z.B. Positioniervorrichtung etc.) zu ermöglichen.

Die Komponenten des Computersystems können kommerziell ausgeführt oder für die jeweilige Implementierung individuell konfiguriert werden. Vorzugsweise verfügt das Computersystem über einen Prozessor, ein Eingabegerät, wie z. B. eine Tastatur oder eine Maus, einen Speicher, wie z. B. eine Festplatte und einen flüchtigen oder nichtflüchtigen Speicher, und Computercode (Software) zur Steuerung der Komponenten des Systems.

Die Steuereinheit kann auch eine programmierbare Leiterplatte (engl. *printed circuit board*), einen Mikrocontroller oder eine andere Vorrichtung zum Empfangen und Verarbeiten von Datensignalen von den Komponenten des Systems umfassen, wie z. B. die Position der Pinzettenarme bzw. Spitzen oder andere sensorische Informationen, beispielsweise über die Position der Embryonen anhand von Kameraaufnahmen.

Die Steuereinheit umfasst vorzugsweise ferner ein computerverwendbares oder computerlesbares Medium, wie z. B. eine Festplatte, einen Direktzugriffsspeicher (RAM), einen Festwertspeicher (ROM), einen Flash-Speicher usw., auf dem eine Computersoftware oder ein Computercode installiert ist. Der Computercode oder die Software zur Durchführung der Steuerung der Komponenten des Systems kann in einer beliebigen Programmiersprache oder modellbasierten Entwicklungsumgebung geschrieben sein, wie z. B., aber nicht beschränkt auf C/C++, C#, Objective-C, Java, Basic/VisualBasic, MATLAB, Simulink, StateFlow, Lab View oder Assembler.

Die Computersoftware und alle funktionalen Beschreibungen der Computersoftware durch Beschreibung der Steuerung bestimmter Geräte oder Aspekte des hierin beschriebenen Systems werden aufgrund einer direkten physischen Ausgabe auf dem System als technische Merkmale betrachtet. Funktionale Beschreibungen der Software können daher als bevorzugte und definierende Ausführungsformen der Erfindung betrachtet werden. Der jeweils verwendete Computercode steht dem Fachmann zur Verfügung und kann mit Standardwissen entsprechend konstruiert werden.

Der Begriff "Steuereinheit ist konfiguriert, für" das Ausführen eines bestimmten Verfahrensschritt, kann eine auf der Steuereinheit installierte Individual- oder Standardsoftware umfassen, die diese Verfahrensschritte initiiert und steuert.

Die Steuereinheit ist dazu eingerichtet, eine wiederholte Ausführung einer Bewegung der Pinzettenarme zum Durchstoßen des Chorions mittels der Spitzen an mindestens drei Punkten in einer ersten Position und einem Aufspreizen der Pinzettenarme in eine zweite Position zur wiederholten Entfernung des Chorion zu gewährleisten.

Zu diesem Zweck kann die Steuereinheit, wie oben beschrieben, eine Computersoftware umfassen, die in Kombination mit den für die Funktion erforderlichen physikalischen Komponenten die notwendigen Schritte ermöglicht. Je nach Ausgestaltung des Systems können diese Schritte eine Translation und/oder Rotation von Pinzettenarmen und/oder eine Translation und/oder Rotation einer Positioniervorrichtung auf dem Embryonen zur Dechorionierung platziert sind umfassen.

Eine vorzugsweise automatisiert steuerbare Halte- oder Positioniervorrichtung zur Positionierung und Ausrichtung der Embryonen gegenüber der Spreizpinzette kann vorzugsweise ebenso Bestandteil des Systems sein.

Bei der Positioniervorrichtung kann es sich beispielsweise um ein Substrat, mit einer linienförmigen Vertiefung handeln, in welche die Embryonen aufgereiht vorliegen. Vorzugsweise ist die Positioniervorrichtung gegenüber der Spreizpinzette translatierbar ausgeführt, sodass sukzessive der Spreizpinzette Embryonen zur Dechorionierung zugeführt werden können.

Alternativ kann die Halte- oder Positioniervorrichtung beispielsweise auch durch ein Zahnrad umgesetzt werden, wobei es bevorzugt ist, dass in den Zwischenräumen der Zähne die Embryonen im Chorion positioniert vorliegen. Durch intermittierende Rotation des Zahnrades kann somit sukzessive ein Embryo nach dem anderen vor einer ortsfesten Spreizpinzette befördert werden, um eine automatisierte Dechorionierung durchzuführen.

Im Folgenden soll die Erfindung an Hand von Beispielen näher erläutert werden, ohne auf diese beschränkt zu sein.

### Kurzbeschreibung der Abbildungen

- Abb. 1: Schematische Illustration und Vergleich von Verfahren zur Entfernung des Chorions eines Embryos gemäß des Standes der Technik a) und bevorzugten Ausführungsformen der Erfindung b) und c).
- Abb. 2: Schematische Illustration einer bevorzugten Ausführungsform einer Spreizpinzetten mit drei Pinzettenarmen an denen endseitig jeweils eine Spitze positioniert vorliegt.
- Abb. 3: Schematische Illustration einer bevorzugte Ausführungsform einer Spreizpinzetten mit zwei Pinzettenarmen an denen endseitig jeweils zwei Spitzen positioniert vorliegen.
- Abb. 4: Schematische Illustration einer bevorzugten Ausführungsformen einer Spreizpinzette, bei der eine besonders gleichmäßig Öffnungsbewegung der Pinzettenarme gewährleistet wird.

### Ausführliche Beschreibung der Abbildungen

Abbildung 1 illustriert schematisch Verfahren zur Entfernung des Chorions **1** eines Embryos gemäß des Standes der Technik a) und bevorzugten Ausführungsformen der Erfindung b) und c).

Bei bekannten mechanischen Verfahren zur Dechorionierung von Zebrafischembryonen wird das Chorion **1** mit zwei feinmechanischen Pinzetten ausgehend von zwei Punkten geöffnet und auseinandergezogen. Alternativ wird das Chorion mit einer feinmechanischen Pinzette mit beiden Spitzen an zwei eng benachbarten Punkten **2** durchstochen und durch Spreizung eine Öffnung **3** geschaffen, durch welche der Embryo austreten kann.

Nachteilig an dem bekannten Verfahren sind mögliche Verletzungen des Embryos während dieser durch die schmale, schlitzförmige Öffnung **3** tritt, sodass die bekannten Verfahren ein hohes Maß an Übung erfordern und zudem nicht in nahliegender Weise automatisierbar sind.

Das erfindungsgemäße Verfahren sieht demgegenüber ein Durchstoßen des Chorion 1 an mindestens drei Punkten **2** vor, wie in der Abbildung 1 b) und c) beispielhaft illustriert.

In Abbildung 1 b) wird das Chorion **1** mittels einer Spreizpinzette mit drei Pinzettenarmen und endseitigen Spitzen an drei Punkten **2** durchstoßen, welche ein kleines Dreieck bilden. Durch anschließendes Aufspreizen der Pinzettenarme bildet sich zwischen den drei Punkten eine polygonale Öffnung **4** in Form eines Dreiecks. Der Austritt des Embryos aus der polygonalen Öffnung **4** ist im Gegensatz zur schlitzförmigen Öffnung **3** deutlich erleichtert.

In Abbildung 1 c) wird das Chorion **1** mittels einer Spreizpinzette mit zwei Pinzettenarmen und jeweils zwei endseitigen Spitzen an vier Punkten **2** durchstoßen, welche ein kleines Rechteck bilden. Durch anschließendes Aufspreizen der Pinzettenarme bildet sich zwischen den vier Punkten eine polygonale Öffnung **4** in Form eines Rechteckes, wobei sich eine Länge des Rechteckes durch das Aufspreizen vergrößert. Durch die vergrößerte polygonale Öffnung **4** kann der Embryo unversehrt austreten.

Das erfindungsgemäße Verfahren kann selbst von unerfahrenen Mitarbeitern bereits nach wenigen Übungen durchgeführt werden und ist gegenüber bekannten Verfahren äußerst zeiteffizient, sicher und zudem für eine Automatisierung geeignet.

Abbildung 2 illustriert eine bevorzugte Ausführungsform einer Spreizpinzette **5** mit drei Pinzettenarmen **6** an denen endseitig jeweils eine Spitze **7** positioniert vorliegt. Die drei Pinzettenarme **6** und die endseitigen Spitzen **7** sind derart angeordnet, dass diese vor dem Aufspreizen im Wesentlichen in einer Ebene vorliegen, um ein gleichzeitiges Durchstoßen eines Chorions an drei Punkten zu gewährleisten. Die Spitzen liegen zu diesem Zweck nicht auf einer Linie kollinear vor, sondern bilden ein Dreieck, bevorzugt ein im Wesentlichen gleichseitiges Dreieck.

Die Pinzettenarme **6** sind elastisch federnde Schenkel, welche an ihrem einen Ende **8** starr verbunden sind, wobei Pinzettenarme **6** durch Ausübung eines Druckes in eine erste Position gebracht werden können, um ein Chorion an drei Punkten in Form eines kleinen Dreieckes zu durchstoßen (vgl. Abb. 1 b, Mitte). Bei Entfernung des Druckes werden die Pinzettenarme **6** und mithin die Spitzen **6** elastisch federnd eine Aufspreizbewegung in eine zweite Position vollziehen, bei der ein vergrößerte polygonale Öffnung in Form eines Dreieckes für den Austritt des Embryos bereitgestellt wird (vgl. Abb. 1 b, rechts).

Abbildung 3 illustriert eine bevorzugte Ausführungsform einer Spreizpinzette **5** mit zwei Pinzettenarmen **6** an denen endseitig jeweils zwei Spitzen **7** positioniert vorliegt. Die zwei Pinzettenarme **6** und die endseitigen Spitzen **7** sind derart angeordnet, dass diese vor dem Aufspreizen im Wesentlichen in einer Ebene vorliegen, um ein gleichzeitiges Durchstoßen eines Chorions an vier Punkten zu gewährleisten. Die mindestens vier Spitzen liegen zu diesem Zweck nicht auf einer Linie kollinear vor, sondern bilden ein Viereck, bevorzugt ein Rechteck.

Die Pinzettenarme **6** sind elastisch federnde Schenkel, welche an ihrem einen Ende **8** starr verbunden sind, wobei die Pinzettenarme **6** durch Ausübung eines Druckes in eine erste Position gebracht werden können, um ein Chorion an vier Punkten in Form eines kleinen Rechteckes zu durchstoßen (vgl. Abb. 1 c, mitte). Bei Entfernung des Druckes werden die beiden Pinzettenarme **6** und mithin die Spitzen **7** elastisch federnd eine Aufspreizbewegung in eine zweite Position vollziehen, bei sich eine Seite des Rechteckes stetig vergrößert. Nach Vollzug der Aufspreizbewegung steht eine vergrößerte polygonale Öffnung in Form eines Rechteckes bereit, welche einen leichten und sicheren Austritt für den Embryo gewährleistet (vgl. Abb. 1c, rechts).

Abbildung 4 illustriert eine bevorzugte Ausführungsform einer Spreizpinzette **5** mit drei Pinzettenarmen **6** an denen endseitig jeweils eine Spitze **7** positioniert vorliegt. Die drei Pinzettenarme **6** liegen endseitig in einem Griff **9** stabilisiert vor und werden zudem durch ein Zwischenstück **10** mit Führungsbohrungen **11** geführt. Am Eintrittsende des Zwischenstückes **10** sind die Führungsbohrungen **11** kongruent zur Stabilisierung der Pinzettenarme **6** im Griff **9** angeordnet, sodass die Pinzettenarme **6** zwischen dem Griff **9** und dem Zwischenstück **10** im Wesentlichen parallel verlaufen. Innerhalb des Zwischenstückes **10** verlaufen die Führungsbohrungen **11** von einer äußeren radialen Position am Eintrittsende zu einer inneren radialen Position, sodass die Pinzettenarme **6** innerhalb des Zwischenstückes **10** aufeinander zu bzw. in Richtung der Zentralachse geführt bzw. gebogen werden.

Durch eine Veränderung des Abstandes zwischen dem Zwischenstückes **10** und dem Griff **9** kann eine besonders gleichmäßiges Aufspreizen der Spitzen **7** erzielt werden.

Fig. 4a illustriert eine erste Position, in welcher die Spitzen **7** nahe beieinander vorliegen und das Chorion an drei Punkten durchstoßen können. Durch Vergrößerung des Abstandes zwischen dem Zwischenstück **10** und dem Griff **9** erfolgt eine Aufspreizen der Pinzettenarme **6** und mithin der Spitzen **7** in eine zweite Position, sodass zwischen den drei Punkten eine polygonale Öffnung in dem Chorion gebildet wird, aus welcher der Embryo heraustreten kann. Die zweite Position ist in der Fig. 4b illustriert.

### Bezugszeichenliste

- 1: Chorion
- 2: Punkte an denen das Chorion durchstoßen wird
- 3: Riss bzw. schmale, schlitzförmige Öffnung
- 4: Öffnung, bevorzugt polygonale Öffnung
- 5: Spreizpinzette
- 6: Pinzettenarme
- 7: Spitzen der Pinzette
- 8: Ende der Spreizpinzette an dem Pinzettenarme als elastische Schenkel starr verbunden vorliegen
- 9: Griff bzw. Halterung für Pinzettenarme
- 10: Zwischenstück
- 11: Führungsbohrungen

### LITERATUR

Čulić-Viskota J. et al. (2012). Surface functionalization of barium titanate SHG nanoprobes for in vivo imaging in zebrafish. Nature Protocols Vol. 7 No. 9, pages 1618-1633.
Cully M. (2019). Zebrafish earn their drug discovery stripes. Nature reviews. Drug discovery, 18(11), 811-813. https://doi.org/10.1038/d41573-019-00165-x
Dooley K., L Zon, Zebrafish: a model system for the study of human disease", Current Opinion in Genetics & Development, Vol. 10, Issue 3, 1 June 2000, Pages 252-256
Henn K., Braunbeck T. Dechorionation as a tool to improve the fish embryo toxicity test (FET) with the zebrafish (Danio rerio) Comp Biochem Physiol C Toxicol Pharmacol. 2011 Jan; 153(1):91-8.
MacRae CA, Peterson RT. Zebrafish as tools for drug discovery. Nat Rev Drug Discov. (2015) 14:721-31. doi: 10.1038/nrd4627
Mandrell David, Lisa Truong, Caleb Jephson, Mushfiqur R. Sarker, Aaron Moore,Christopher Lang1, Michael T. Simonich, and Robert L. Tanguay, Automated Zebrafish Chorion Removal and Single EmbryoPlacement: Optimizing Throughput of Zebrafish DevelopmentalToxicity Screens J Lab Autom. 2012 February ; 17(1): 66-74.
Parng C, Seng WL, Semino C, McGrath P. Zebrafish: a preclinical model for drug screening. Assay Drug Dev Technol. 2002 Nov;1(1 Pt 1):41-8. doi: 10.1089/154065802761001293. PMID: 15090155.
Rennekamp AJ, Peterson RT. 15 years of zebrafish chemical screening. Curr Opin Chem Biol. (2015) 24:58-70. doi: 10.1016/j.cbpa.2014.10.025
Steven Cassar, Isaac Adatto, Jennifer L. Freeman,§ Joshua T. Gamse Iñaki Iturria, Christian Lawrence, Arantza Muriana, Randall T. Peterson,∇ Steven Van Cruchten, and Leonard I. Zon: Use of Zebrafish in Drug Discovery Toxicology, Chem. Res. Toxicol. 2020, 33, 95-118.

## Patentansprüche

1. Verfahren zur Entfernung des Chorions (1) eines nicht menschlichen Embryos umfassend die Schritte
a) Bereitstellung einer Spreizpinzette (5) umfassend mindestens drei Spitzen (7), welche endseitig an Pinzettenarmen (6) vorliegen
b) Durchstoßen des Chorions (1) an mindestens drei Punkten mittels der Spitzen (7)
c) Aufspreizen der Pinzettenarme (6), sodass sich zwischen den mindestens drei Punkten (2) eine Öffnung (4) in dem Chorion (1) bildet, aus welcher der Embryo heraustreten kann.

2. Verfahren gemäß dem vorherigen Anspruch
**dadurch gekennzeichnet, dass**
der Embryo ein Fischembryo, bevorzugt ein Zebrafischembryo, Killifisch oder Medakaembryo ist.

3. Verfahren gemäß einem oder mehreren der vorherigen Ansprüche
**dadurch gekennzeichnet, dass**
die mindestens drei Punkte (2), an denen das Chorion (1) durchstoßen wird nicht auf einer Linie liegen, sondern ein Polygon aufspannen.

4. Verfahren gemäß einem oder mehreren der vorherigen Ansprüche
**dadurch gekennzeichnet, dass**
die Spreizpinzette (5) mindestens drei Pinzettenarme (6) umfasst, an welchen jeweils endseitige Spitzen (7) vorliegen, und das Chorion (1) an mindestens drei Punkten (2) durchstoßen wird, welche ein Dreieck bilden, bevorzugt ein im Wesentlichen gleichseitiges Dreieck.

5. Verfahren gemäß einem oder mehreren der vorherigen Ansprüche 1- 3
**dadurch gekennzeichnet, dass**
die Spreizpinzette (5) mindestens zwei Pinzettenarme (6) umfasst an denen endseitig jeweils zwei Spitzen (7) vorliegen und das Chorion (1) an vier Punkten durchstoßen wird, welche ein Viereck bilden und wobei bevorzugt durch das Aufspreizen der zwei Pinzettenarme (6) eine Länge des Viereckes vergrößert wird.

6. Verfahren gemäß einem oder mehreren der vorherigen Ansprüche
**dadurch gekennzeichnet, dass**
die Pinzettenarme (6) automatisiert geführt werden, wobei insbesondere das Durchstoßen des Chorion (1) und Aufspreizen der Pinzettenarme (6) zur Entfernung des Chorion automatisiert erfolgt.

7. Hochdurchsatz-Verfahren zur automatisierten Testung der Wirkung von Substanzen auf nicht menschliche Embryonen, bevorzugt Zebrafisch- oder Medakaembryonen
**dadurch gekennzeichnet, dass**
eine Entfernung des Chorions (1) der Embryonen mittels eines Verfahrens gemäß der vorherigen Ansprüche durchgeführt wird.

8. Spreizpinzette (5) für ein Verfahren zur Entfernung eines Chorions (1) gemäß einem oder mehreren der vorherigen Ansprüche
umfassend mindestens drei Spitzen (7), welche endseitig an Pinzettenarmen (6) vorliegen,
wobei die Pinzettenarme (6) für eine Bewegung in mindestens zwei Positionen konfiguriert sind, sodass die mindestens drei Spitzen (7) in einer ersten Position ein Chorion (1) in mindestens drei Punkten (2) durchstoßen können und wobei ferner durch ein Aufspreizen der Pinzettenarme (7) in eine zweite Position, sich zwischen den mindestens drei Punkten (2) eine polygonale Öffnung (4) in dem Chorion (1) bildet, aus welcher der Embryo heraustreten kann.

9. Spreizpinzette (5) gemäß des vorherigen Anspruches
**dadurch gekennzeichnet, dass**
die Spreizpinzette (5) mindestens drei Pinzettenarme (6) umfasst, an welchen jeweils eine endseitige Spitze (7) vorliegt oder die Spreizpinzette (5) zwei Pinzettenarme (6) umfasst, an welchen jeweils zwei endseitige Spitzen (7) vorliegen.

10. Spreizpinzette (5) gemäß einem der vorherigen Ansprüche 8 oder 9
**dadurch gekennzeichnet, dass**
die Pinzettenarme (6) elastisch federnde Schenkel sind, welche an ihrem einen Ende (8) starr verbunden sind, wobei die Pinzettenarme (6) durch Ausübung eines Druckes in die erste Position gebracht werden können und bei Entfernung des Druckes elastisch federnd eine Aufspreizbewegung in die zweite Position vollziehen.

11. Spreizpinzette (5) gemäß einem der vorherigen Ansprüche 8 oder 9
**dadurch gekennzeichnet, dass**
die Pinzettenarme (6) endseitig in einem Griff (9) stabilisiert vorliegen und in einem Zwischenstück (10) mit Führungsbohrungen (11) auf eine Zentralachse des Zwischenstückes (10) zugeführt werden, sodass durch eine Vergrößerung des Abstandes zwischen Zwischenstückes (10) und Griff (9) die Pinzettenarme (6) von einer ersten Position zu einer zweiten Position aufgespreizt werden.

12. System umfassend
a. eine Spreizpinzette (5) gemäß einem der Ansprüche 8-11 sowie
b. einem Antrieb zur Bewegung der Pinzettenarme (6)
**dadurch gekennzeichnet, dass**
der Antrieb mindestens für eine Bewegung der Pinzettenarme (6) zwischen einer ersten und einer zweiten Position konfiguriert ist.

13. System gemäß dem vorherigen Anspruch
**dadurch gekennzeichnet, dass**
das System eine Steuereinheit zur Regulation des Antriebs umfasst,
wobei die Steuereinheit bevorzugt für eine wiederholte Ausführung einer Bewegung der Pinzettenarme (6) zum Durchstoßen des Chorions (1) mittels der Spitzen (7) an mindestens drei Punkten (2) in einer ersten Position und einem Aufspreizen der Pinzettenarme (6) in eine zweite Position zur wiederholten Entfernung des Chorions (1) einer Vielzahl von Embryonen konfiguriert ist.

## Claims

1. A method for removing the chorion (1) of a non-human embryo, comprising the steps of
a) providing spreading tweezers (5) comprising at least three tips (7), which are disposed at the distal ends of tweezers arms (6)
b) penetrating the chorion (1) at at least three sites by means of the tips (7)
c) spreading the tweezers arms (6) so that an opening (4) is formed between the at least three sites (2) in the chorion (1) from which the embryo can emerge.

2. The method of the preceding claim
**characterized in that**
the embryo is a fish embryo, preferably a zebrafish embryo, killifish or meda embryo.

3. The method of any one or more of the preceding claims
**characterized in that**
the at least three sites (2) at which the chorion (1) is pierced are not in line, but span a polygon.

4. The method of any one or more of the preceding claims
**characterized in that**
the spreading tweezers (5) comprise at least three tweezers arms (6), each of which is provided with a tip (7) at its distal end, and the chorion (1) is pierced at at least three sites (2) which form a triangle, preferably a substantially equilateral triangle.

5. The method of any one or more of the preceding claims 1 to 3
**characterized in that**
the spreading tweezers (5) comprise at least two tweezers arms (6), each of which is provided with two tips (7) at its distal end, and the chorion (1) is pierced at four sites which form a quadrilateral, and wherein a length of the quadrilateral is preferably enlarged by spreading the two tweezers arms (6).

6. The method of any one or more of the preceding claims
**characterized in that**
the tweezers arms (6) are guided automatically, wherein, in particular, the piercing of the chorion (1) and the spreading of the tweezers arms (6) for removing the chorion occur automatically.

7. High-throughput method for automated testing of the effects of substances on non-human embryos, preferably zebrafish or medaca embryos
**characterized in that**
the removal of the chorion (1) of the embryos is carried out by means of a method of the preceding claims.

8. Spreading tweezers (5) for a method for removing a chorionic ion (1) according to any one or more of the preceding claims comprising at least three tips (7), which are disposed at the distal ends of tweezers arms (6),
wherein the tweezers arms (6) are configured for movement in at least two positions, so that the at least three tips (7) in a first position can penetrate a chorion (1) in at least three sites (2), and wherein further, by spreading the tweezers arms (7) into a second position, a polygonal opening (4) in the chorion (1) is formed between the at least three sites (2), from which polygonal opening the embryo can emerge.

9. The spreading tweezers (5) according to the preceding claim
**characterized in that**
the spreading tweezers (5) comprise at least three tweezers arms (6), each of which is provided with one tip (7) at its distal end, or the spreading tweezers (5) comprise two tweezers arms (6), each of which is provided with two tips (7) at its distal end.

10. The spreading tweezers (5) according to any one of the preceding claims 8 or 9
**characterized in that**
the tweezers arms (6) are elastically resilient legs, each of which is rigidly connected at its one end (8), wherein the tweezers arms (6) can be brought into the first position by exerting a pressure and undergo an elastically resilient spreading movement into the second position when the pressure is released.

11. The spreading tweezers (5) according to any one of the preceding claims 8 or 9
**characterized in that**
the tweezers arms (6) are stably supported at their distal ends in a handle (9) and are fed in an intermediate piece (10) with guide bores (11) to a central axis of the intermediate piece (10) so that by increasing the distance between the intermediate piece (10) and the handle (9), the tweezers arms (6) are spread from a first position to a second position.

12. A system comprising
a. spreading tweezers (5) according to any one of claims 8 to 11
and
b. a drive for moving the tweezers arms (6)
**characterized in that**
the drive is at least configured to move the tweezers arms (6) between a first and a second position.

13. The system according to the preceding claim
**characterized in that**
the system comprises a control unit for regulating the drive, wherein the control unit is preferably configured to repeatedly execute a movement of the tweezers arms (6) for piercing the chorion (1) by means of the tips (7) at at least three sites (2) in a first position, and to spread the tweezers arms (6) into a second position for the repeated removal of the chorion (1) from a plurality of embryos.

## Revendications

1. Procédé pour retirer le chorion (1) d'un embryon non humain comprenant les étapes suivantes
a) Mise à disposition d'une pince d'écartement (5) comprenant au moins trois pointes (7) qui se trouvent à l'extrémité sur les bras de pince (6)
b) Perçage du chorion (1) en au moins trois points au moyen des pointes (7)
c) Écartement des bras de pince (6) de sorte que, entre les au moins trois points (2), une ouverture (4) se forme dans le chorion (1), par laquelle l'embryon peut sortir.

2. Procédé selon la revendication précédente
**caractérisé en ce que**
l'embryon est un embryon de poisson, de préférence un embryon de poisson-zèbre, de poisson-killife ou de médaca.

3. Procédé selon l'une ou plusieurs des revendications précédentes
**caractérisé en ce que**
les au moins trois points (2) sur lesquels le chorion (1) est percé ne se trouvent pas sur une ligne, mais s'étendent sur un polygone.

4. Procédé selon l'une ou plusieurs des revendications précédentes
**caractérisé en ce que**
la pince d'écartement (5) comprend au moins trois bras de pince (6) sur lesquels se trouvent des pointes (7) respectives à l'extrémité, et le chorion (1) est percé en au moins trois points (2) qui forment un triangle, de préférence un triangle sensiblement équilatéral.

5. Procédé selon l'une des revendications précédentes 1 à 3
**caractérisé en ce que**
la pince d'écartement (5) comprend au moins deux bras de pince (6), chacun d'eux ayant deux pointes (7) respectives à l'extrémité, et le chorion (1) est percé en quatre points qui forment un quadrilatère, et dans lequel une longueur du quadrilatère est de préférence augmentée par l'écartement des deux bras de pince (6).

6. Procédé selon l'une ou plusieurs des revendications précédentes
**caractérisé en ce que**
les bras de pince (6) sont guidés automatiquement, dans lequel, en particulier, le perçage du chorion (1) et l'écartement des bras de pince (6) pour le retrait du chorion interviennent automatiquement.

7. Procédé à haut débit pour tester automatiquement les effets des substances sur des embryons non humains, de préférence des embryons de poisson zèbre ou de médaka
**caractérisé en ce que**
le retrait du chorion (1) des embryons est effectuée au moyen d'un procédé selon l'une quelconque des revendications précédentes.

8. Pince d'écartement (5) pour un procédé de retrait d'un chorion (1) selon l'une ou plusieurs des revendications précédentes
comprenant au moins trois pointes (7) qui se trouvent à l'extrémité sur les bras de pince (6),
dans laquelle les bras de pince (6) sont configurés pour se déplacer dans au moins deux positions, de sorte que les au moins trois pointes (7) peuvent percer un chorion (1) en au moins trois points (2) dans une première position, et en outre dans laquelle, par un écartement des bras de pince (7) dans une seconde position, une ouverture (4) polygonale est formée dans le chorion (1) entre les au moins trois points (2), ouverture à partir de laquelle l'embryon peut émerger.

9. Pince d'écartement (5) selon la revendication précédente
**caractérisé en ce que**
la pince d'écartement (5) comprend au moins trois bras de pince (6) sur lesquels se trouve une pointe (7) respective à l'extrémité ou la pince d'écartement (5) comprend deux bras de pince (6) sur lesquels se trouvent deux pointes (7) respectives à l'extrémité.

10. Pince d'écartement (5) selon l'une des revendications 8 ou 9 précédentes
**caractérisé en ce que**
les bras de pince (6) sont des branches à ressort élastique qui sont reliées rigidement à une extrémité (8), dans laquelle les bras de pince (6) peuvent être amenés dans la première position par l'exercice d'une pression et, lorsque la pression est retirée, effectuent un mouvement d'écartement à ressort élastique dans la seconde position.

11. Pince d'écartement (5) selon l'une des revendications 8 ou 9 précédentes
**caractérisé en ce que**
les bras de pince (6) sont stabilisés à l'extrémité dans un manche (9) et sont guidés dans une pièce intermédiaire (10) avec des trous de guidage (11) sur un axe central de la pièce intermédiaire (10), de sorte que, par une augmentation de la distance entre la pièce intermédiaire (10) et le manche (9), les bras de pince (6) sont écartés d'une première position à une seconde position.

12. Système comprenant
a. une pince d'écartement (5) selon l'une quelconque des revendications 8 à 11
ainsi
b. qu'un mécanisme d'entraînement pour déplacer les bras de pince (6)
**caractérisé en ce que**
l'entraînement est configuré au moins pour un mouvement des bras de pince (6) entre une première et une seconde position.

13. Système selon la revendication précédente
**caractérisé en ce que**
le système comprend une unité de commande pour la régulation de l'entraînement,
dans lequel l'unité de commande est de préférence configurée pour une exécution répétée d'un mouvement des bras de pince (6) pour percer le chorion (1) au moyen des pointes (7) en au moins trois points (2) dans une première position et un écartement des bras de pince (6) dans une seconde position pour le retrait répété du chorion (1) d'une pluralité d'embryons.
